# EUROPEAN PATENT APPLICATION

(11) **EP 4 818 144 A1**
(43) Date of publication of application: **07.10.2026**
(21) Application number: 25784244.3
(22) Date of filing: 07.03.2025
(51) Int. Cl.: C07C 13/18

(54) **METHOD FOR PRODUCING CYCLOHEXANONE AND CYCLOHEXANOL BY CYCLOHEXANE OXIDATION**

(30) Priority: 10.02.2025 CN 202510143958
(71) Applicant: Changsha Xinghe New Materials Co., Ltd., Changsha, Hunan 410003 (CN)
(72) Inventor: XIAO, Youchang, Changsha, Hunan 410003 (CN); XIAO, Zaosheng, Changsha, Hunan 410003 (CN)
(74) Representative: Sun, Yiming
(86) International application number: PCT/CN2025/081187
(87) International publication number: WO 2026/165974

(57) **Abstract**

A method for producing cyclohexanol and cyclohexanone by oxidizing cyclohexane comprises the steps of: (1) oxidizing cyclohexane with molecular oxygen under non-catalytic conditions to obtain an oxidation liquid containing cyclohexyl hydroperoxide; (2) adding a cyclohexyl hydroperoxide decomposition catalyst and a scale inhibitor to the obtained oxidation liquid for multi-effect reaction distillation, to decompose the cyclohexyl hydroperoxide into cyclohexanol and cyclohexanone, and at the same time, to recover the cyclohexane unreacted in step (1) and return the cyclohexane to step (1); and (3) adding an alkali liquid to a decomposition liquid containing 50%-70% of cyclohexane, as obtained after the distillation, for a neutralization and saponification reaction, to convert the remaining cyclohexyl hydroperoxide completely into cyclohexanol and cyclohexanone to obtain an alcohol-ketone mixture. The present invention combines the decomposition of cyclohexyl hydroperoxide and the recycling of cyclohexane into one step, eliminating the need for a large-scale alkali liquid separation process, which greatly improves the production efficiency and reduces the production costs.

## Description

### TECHNICAL FIELD

The present invention relates to a method for preparing organic compounds cyclohexanone and cyclohexanol, and in particular to a method for producing cyclohexanone and cyclohexanol with cyclohexane as a starting material.

### DESCRIPTION OF RELATED ART

Cyclohexanone, an important organic chemical raw material, is mainly used as an intermediate for the production of caprolactam, as well as adipic acid and its salts. Due to its characteristics such as good solubility, low toxicity and relatively low price, cyclohexanone can also be used as an excellent solvent, as well as a solubilizer for paints and inks.

The production methods for cyclohexanone include a phenol hydrogenation method, a cyclohexane oxidation method, and a cyclohexene hydration method. Although the cyclohexene hydration method has become the method with the largest production capacity at present, the cyclohexane oxidation method remains vital in addressing the cyclohexane, byproduct during producing cyclohexene. It can complement the cyclohexanone device which using the cyclohexene hydration method ,enable mutual supply of raw materials to form a closed-loop circular economic chain, and allow for participation in market competition with superior quality and lower production costs.

Existing cyclohexane oxidation processes are mainly classified into a catalytic oxidation process and a non-catalytic oxidation process.

The catalytic cyclohexane oxidation process is invented by DuPont in the United States, in which a cobalt salt is used to catalyze the oxidation of cyclohexane; the oxidation reaction of cyclohexane and the decomposition reaction of an intermediate product cyclohexyl hydroperoxide occurs in an oxidation reactor; and after alkali decomposition and the recovery by a cyclohexane recovery system, a mixture of cyclohexanone and cyclohexanol is obtained. However, in the catalytic oxidation process, the cyclohexane conversion rate is 5% and the yield of cyclohexanone and cyclohexanol is only 70%-75%, since the cyclohexanone produced by the decomposition of cyclohexyl hydroperoxide is more liable to be oxidated by molecular oxygen than cyclohexane in the oxidation reactors.

In recent years, a biomimetic catalysis process for cyclohexane has been proposed, in which metalloporphyrins and other catalysts are used to catalyze the air oxidation of cyclohexane. The biomimetic catalytic oxidation is declared to increase the single-pass cyclohexane conversion rate to 8%. However, it fails to solve the core problem that the decomposed products cyclohexanol and cyclohexanone are more liable to oxidation than cyclohexane. As a result, serious deep oxidation is caused during the oxidation process, and the yields of cyclohexanone and cyclohexanol are low, which are less than 70%.

There are two main routes for the non-catalytic cyclohexane oxidation process. One route is developed by Rhodia in France, in which depleted oxygen is used for the oxidation of cyclohexane without addition of a catalyst, and cyclohexyl hydroperoxide in the oxidation liquid is subjected to a homogeneous decomposition process using tert-butyl chromate as a catalyst; and the other route is developed by DSM in the Netherlands, in which air is used for the non-catalytic oxidation of cyclohexane, and cyclohexyl hydroperoxide is subjected to a heterogeneous catalytic decomposition process using cobalt acetate in an alkaline aqueous of sodium hydroxide solution. The innovative point of the two routes lies in separating the oxidation reaction from the decomposition reaction, by which the single-pass oxidation conversion rate of cyclohexane is reduced to 3.5%, and cyclohexyl hydroperoxide is mainly produced while the formation of cyclohexanol and cyclohexanone in the oxidized environment is reduced, such that the deep oxidation is reduced and the oxidation yield is improved. However, the low conversion rate of oxidation leads to larger cyclohexane circulation, making the matched alkali decomposition system and cyclohexane recovery system bottlenecks of the entire system. At present, the production capacity of the largest single-line DSM-imitated cyclohexane oxidation device is only 100,000 tons per year.

In technical solutions reported by CN201210085933.6 and CN201210091366.5, the homogeneous decomposition technology of Rhodia in France and the alkali decomposition technology of DSM in the Netherlands are combined to fully utilize the high selectivity of homogeneous decomposition and the high conversion rate characteristics of alkali decomposition, allowing the process of preparing cyclohexanol and cyclohexanone by oxidizing cyclohexane to develop to a new stage. In this way, the specific consumptions of both cyclohexane and caustic soda are greatly reduced, and the most market-competitive technology of preparing cyclohexanol and cyclohexanone by oxidizing cyclohexane is developed. Although this technology recovers part of the cyclohexane during the homogeneous decomposition process, the flow of the organic phase in the alkali decomposition process remains large, and the alkali separation load remains a major obstacle for the continued capacity expansion of the plant. Furthermore, a set of cyclohexane recovery columns still needs to consume a large amount of steam to recover cyclohexane. At present, the production capacity of the largest single-line cyclohexane oxidation plant using this technology can only reach 120,000 tons per year, and it cannot be further expanded.

### BRIEF SUMMARY OF THE INVENTION

The technical problem to be solved by the present invention is to provide a method for producing cyclohexanone and cyclohexanol by oxidizing cyclohexane with shorter process, lower steam consumption, lower raw material costs, and greater production capacity of single-line cyclohexane oxidation devices.

The present invention solves the technical problems by using a technical solution as follows: a method for producing cyclohexanone and cyclohexanol by oxidizing cyclohexane, including the steps of:
(1) oxidizing cyclohexane with molecular oxygen under non-catalytic conditions to obtain an oxidation liquid containing cyclohexyl hydroperoxide,
   wherein oxidizing under the non-catalytic conditions includes subjecting liquid cyclohexane and the molecular oxygen to perform an oxidation reaction in an oxidation reaction device without addition of a catalyst, to generate the oxidation liquid with an intermediate cyclohexyl hydroperoxide as a main component; and
(2) adding a cyclohexyl hydroperoxide decomposition catalyst and a scale inhibitor to the obtained oxidation liquid from step (1) for multi-effects reaction distillation, to decompose more than 95% of the cyclohexyl hydroperoxide into cyclohexanol and cyclohexanone, and at the same time, to recover the cyclohexane unreacted in step (1) during the distillation and return the cyclohexane to step (1) to serve as a starting material for the oxidation reaction in step (1),
   wherein the multi-effect reaction distillation of the oxidation liquid includes: subjecting the oxidation liquid containing the cyclohexyl hydroperoxide to a decomposition reaction in a distillation column under actions of the decomposition catalyst and the scale inhibitor, and at the same time, condensing and circulating most of the cyclohexane unoxidized in step (1) from a top of the distillation column to an oxidation reactor in step (1) by means of multi-effects distillation using sensible heat of the oxidation liquid, decomposition heat and auxiliary heat, while discharging a small amount of acid water, and a bottom discharge after distillation includes a decomposition liquid containing 50%-70% of cyclohexane, and balance of cyclohexanone, cyclohexanol and a trace amount of cyclohexyl hydroperoxide; and
(3) adding an alkali liquid and water to the decomposition liquid containing the 50%-70% of cyclohexane, as obtained from step (2), for a neutralization and saponification reaction, to convert the remaining cyclohexyl hydroperoxide completely into cyclohexanol and cyclohexanone to obtain an alcohol-ketone mixture,
   wherein the neutralization and saponification reaction includes: removing most of acids from the decomposition liquid containing the 50%-70% of cyclohexane by water washing to separate acid water, treating the organic phase by the alkali liquid to neutralize acids and saponify esters in this crude alcohol-ketone mixture and to convert the remaining cyclohexyl hydroperoxide completely into cyclohexanol and cyclohexanone, separating a water phase from a saponification liquid, and then drying the organic phase of the saponification liquid to remove cyclohexane and water to obtain a mixture of cyclohexanone and cyclohexanol, and the cyclohexane removed by drying the organic phase is returned to step (1) to serve as a starting material for the oxidation reaction in step (1).

Further, in step (1), the oxidation reaction device is formed by connecting no fewer than three oxidation reactors in series; and a reaction temperature is controlled to 160°C-170°C, and a molar conversion rate of the cyclohexane is controlled to 2.5%-4.0%, preferably 3.0-3.5%. The use of the oxidation reaction device formed by connecting no fewer than three oxidation reactors in series to perform oxidation of cyclohexane with molecular oxygen under non-catalytic conditions is to achieve a reaction form similar to a piston flow, thereby improving the reaction efficiency.

Further, in step (2), the cyclohexyl hydroperoxide decomposition catalyst is an oil-soluble transition metal catalyst, preferably a transition metal salt catalyst or transition metal acid ester compound of chromium, cobalt, iron, manganese, molybdenum, vanadium or the like.

Further, the oil-soluble transition metal acid ester compound is tert-butyl chromate.

Further, in step (2), during the distillation, the cyclohexyl hydroperoxide undergoes a catalytic decomposition reaction, from which reaction heat is used to evaporate the cyclohexane, and a decomposition rate of the cyclohexyl hydroperoxide is controlled to 93%-98%, preferably 95%-97%.

Further, in step (2), the scale inhibitor is an HEDP ester, i.e., 1-hydroxyethylidene-1,1-diphosphonate ester. The scale inhibitor functions to prevent the precipitation of organic acid transition metal salts from blocking the reactors and pipelines. The HEDP ester also has the function of a dispersant. That is, the HEDP ester acts as both a scale inhibitor and a dispersant and exhibits both the functions of scale inhibition and dispersion.

Further, in step (2), the multi-effects reaction distillation of the oxidation liquid is performed in a multi-stage distillation column, preferably, a three-stage distillation column, in which case a first-stage distillation column has a top operating pressure of 0.20 MpaA-0.35 MpaA (with A representing absolute pressure among the pressure units used in the chemical engineering literature), a top operating temperature of 105°C-130°C, a bottom operating pressure of 0.22 MPaA-0.4 MPaA, and a bottom operating temperature of 110°C-135°C; a second-stage distillation column has a top operating pressure of 0.10 MpaA-0.24 MpaA, a top operating temperature of 85°C-110°C, a bottom operating pressure of 0.12 MPaA-0.25 MPaA, and a bottom operating temperature of 90°C-105°C; and a third-stage distillation column has a top operating pressure of 0.04 MpaA-0.06 MpaA, a top operating temperature of 55°C-70°C, a bottom operating pressure of 0.05 MPaA-0.07 MPaA, and a bottom operating temperature of 65°C-80°C, and the decomposition liquid discharged from a bottom of the third-stage distillation enters an secondary column of the third-stage distillation and is fine-tuned using a small amount of steam to a cyclohexane content of 50%-70%, preferably 60%-65%.

Further, the three-stages distillation has a specific process flow as follows: The oxidation liquid from the non-catalytic cyclohexane oxidation reaction device exchanges heat with the cyclohexane, is then mixed with the scale inhibitor and the decomposition catalyst, and enters the bottom of the first distillation column for cyclohexane, where the lower part of the first distillation column is provided with an internal diversion cylinder; during the decomposition reaction of cyclohexyl hydroperoxide, the cyclohexane is evaporated by taking flash evaporation under reduced pressure, reaction heat, and the steam reboiler as heat sources for the first distillation column; gas-phase cyclohexane in the first distillation column enters a reboiler of the second distillation column; and a bottom liquid in the first distillation column enters the bottom of the second distillation column. Condensed cyclohexane from the reboiler of the second distillation column enters a cyclohexane condensate tank, exchanges heat with the oxidation liquid after pressurization via a cyclohexane pump, and is then delivered to an oxidation reaction procedure. Gas-phase cyclohexane in the second distillation column enters a reboiler of a third distillation column for heat exchange, condensed cyclohexane enters a condensate tank, and cyclohexane condensate is pressurized by the cyclohexane pump and then enters a cyclohexane-water separator. A bottom liquid in the second distillation column enters the bottom of the third distillation column; gas-phase cyclohexane in the third distillation column enters a condenser; a bottom liquid in the third distillation column enters a secondary distillation column of the third stage distillation; and a bottom liquid in the secondary distillation column is pressurized by the decomposition liquid discharge pump and then delivered to a neutralization and saponification procedure. A non-condensable gas from the outlet of a vacuum pump enters a tail gas absorption device, and the cyclohexane condensate enters a cyclohexane-water separator. Part of the cyclohexane from the cyclohexane-water separator refluxes, the remaining cyclohexane is pressurized via the cold cyclohexane pump and then delivered to an oxidation heat recovery section, and the separated acid water is subjected to an acid water treatment procedure.

Further, in step (3), the alkali liquid is an aqueous NaOH solution; and a neutralization and saponification process includes: washing the decomposition liquid with water, subjecting the water-washed decomposition liquid and the aqueous NaOH solution to a neutralization and saponification reaction in a reactor, separating a saponification liquid, removing cyclohexane and water in a drying column to obtain an alcohol-ketone mixture with a purity of more than 97%.

Further, in step (3), in the neutralization and saponification process, an ion concentration of OH- in the alkali liquid is 0.3 Mol/L-1.5 Mol/L, preferably 0.5 Mol/L-1.0 Mol/L.

Further, in step (3), a reaction temperature for the neutralization and saponification is controlled to 80°C-110°C, preferably 90°C-100°C.

Compared with the prior art, the present invention combines the decomposition of cyclohexyl hydroperoxide and the recovery of cyclohexane into one step creatively, reduces the burden in alkali liquid separation by a factor of 10, and eliminates the need of building a large-scale cyclohexane recovery device, making it possible to increase the production capacity of the designed single cyclohexane oxidation plant to 300,000 tons per year. Meanwhile, most of the cyclohexyl hydroperoxide is decomposed under homogeneous conditions, leading to higher system yield and minimum unit consumptions of cyclohexane, caustic soda (sodium hydroxide) and steam, which greatly improves the production efficiency and reduces the production costs.

### BRIEF DESCRIPTION OF THE SEVERAL VIEWS OF THE DRAWINGS

FIG. 1 shows a schematic diagram of a process flow of a method for preparing cyclohexanone and cyclohexanol by oxidizing cyclohexane according to an embodiment; and
FIG. 2 shows a schematic diagram of a structure and working process flow of a three-effect reaction distillation system for an oxidation liquid used in an embodiment of the method of the present invention.

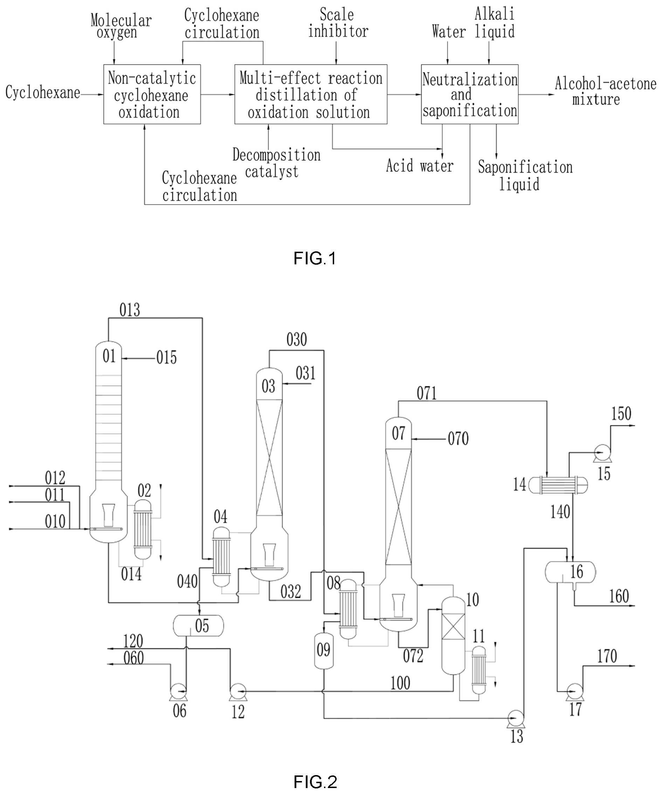

In FIG. 2: 01-first distillation column for cyclohexane, 02-steam reboiler of first distillation column, 03-second distillation column for cyclohexane, 04-reboiler of second distillation column, 05-condensate tank of reboiler of second distillation column, 06-first cyclohexane pump, 07-third distillation column for cyclohexane, 08-reboiler of third distillation column, 09-condensate tank of reboiler of third distillation column, 10-secondary distillation column of the third stage distillation for cyclohexane, 11-steam reboiler of the secondary distillation column of the third stage distillation for cyclohexane, 12-decomposition liquid discharge pump, 13-second cyclohexane pump, 14-condenser, 15-vacuum pump, 16-cyclohexane-water separator, 17-cold cyclohexane pump;
010-oxidation liquid, 011-scale inhibitor, 012-decomposition catalyst, 013-gas-phase cyclohexane in first distillation column, 014-bottom liquid in first distillation column, 015-reflux liquid in first distillation column, 030-gas-phase cyclohexane in second distillation column, 031-reflux liquid in second distillation column, 032-bottom liquid in second distillation column, 040-cyclohexane condensate in first distillation column, 060-cyclohexane condensate in second distillation column, 070-reflux liquid in third distillation column, 071-gas-phase cyclohexane in third distillation column, 072-bottom liquid in third distillation column, 100-bottom liquid in secondary distillation column of the third stage distillation for cyclohexane, 120-decomposition liquid to be neutralized and saponified, 140-cyclohexane condensate in third distillation column, 150-noncondensable gas, 160-acid water, 170- cold cyclohexane delivered to oxidation.

### DETAILED DESCRIPTION OF THE INVENTION

The present invention will be further illustrated below in conjunction with the examples and the accompanying drawings.

### Example

Referring to FIGS. 1 and 2, the method for producing cyclohexanone and cyclohexanol by oxidizing cyclohexane in an example includes operational steps (with the material balance data based on a production line of cyclohexane oxidation device of 120,000 tons per year as an example) as follows.
(1) Cyclohexane is oxidized with molecular oxygen under non-catalytic conditions to obtain an oxidation liquid containing cyclohexyl hydroperoxide.
   Fresh cyclohexane (14,550 kg/h), circulating cyclohexane (509,582 kg/h) and compressed air (20,388 Nm³/h) with the molar fraction of oxygen being 21% are fed to an oxidation reaction device for non-catalytic oxidation. The reaction temperature is controlled to 163°C-170°C, the reaction pressure is controlled to 1.32 MPaA, the gas-phase discharge from the top of the oxidation reaction device is controlled at 108,418 kg/h, the cyclohexane oxidation liquid discharged at the bottom is controlled at 421,111 kg/h, and the concentrations of cyclohexyl hydroperoxide, cyclohexanol, cyclohexanone, cyclohexane and other acid and ester impurities in the oxidation liquid are 3.2%wt, 0.68%wt, 0.34%wt, 95.36%wt, and 0.42%wt, respectively.
(2) A catalyst for cyclohexyl hydroperoxide decomposition and a scale inhibitor are added to the obtained oxidation liquid from step (1) for multi-effect reaction distillation, to decompose the cyclohexyl hydroperoxide into cyclohexanol and cyclohexanone, and at the same time, the cyclohexane unreacted in step (1) is recovered during the distillation and returned to step (1) to serve as a starting material for the oxidation reaction in step (1).

The oxidation liquid (421,111 kg/h) at 163°C from the non-catalytic cyclohexane oxidation reactor exchanges heat with cyclohexane 060 at 105°C to cool down to 155°C, is then mixed with the decomposition catalyst tert-butyl chromate 012 (2.2 kg/h) and the scale inhibitor 1-hydroxyethylidene-1,1-diphosphonate 011 (4.5 kg/h), and enters the bottom of the first distillation column for cyclohexane 01. A first distillation column for cyclohexane 01 has a top operating pressure of 0.23 MPaA, a top operating temperature of 110°C, a bottom operating pressure of 0.26 MPaA, and a bottom operating temperature of 115°C. The lower part of the first distillation column is provided with an internal diversion cylinder; during the decomposition reaction of the cyclohexyl hydroperoxide, the cyclohexane is evaporated by taking flash evaporation under reduced pressure, reaction heat, and the steam reboiler 02 as heat sources for the first distillation column 01; and gas-phase cyclohexane 013 in the first distillation column as a heat source enters a reboiler 04 of a second distillation column 07, and a bottom liquid 014 in the first distillation column enters the bottom of the second distillation column 07.

The cyclohexane (178,518 kg/h) cooled to 105°C in the reboiler 04 of the second distillation column enters a cyclohexane condensate tank 05, is pressurized by a cyclohexane pump 06, exchanges heat with the oxidation liquid to reach 120°C, and then is delivered to an oxidation reaction procedure. The second distillation column for cyclohexane has a top operating pressure of 0.11 MPaA, a top operating temperature of 85°C, a bottom operating pressure of 0.13 MPaA, and a bottom operating temperature of 90°C. The bottom liquid 014 in the first distillation column enters an internal diversion cylinder in the bottom of the second distillation column 03; during the decomposition reaction of the cyclohexyl hydroperoxide, the cyclohexane is evaporated by taking flash evaporation under reduced pressure, reaction heat, and the reboiler 04 as heat sources for the second distillation column 03; gas-phase cyclohexane 030 in the second distillation column as a heat source enters a reboiler 08 of a third distillation column for heat exchange; the cyclohexane cooled to 75°C enters a condensate tank 09; and the cyclohexane condensate is pressurized by a cyclohexane pump 13 and then enters a cyclohexane-water separator 16.

A bottom liquid 032 in the second distillation column enters an internal diversion cylinder in the bottom of the third distillation column 07 for the decomposition reaction of cyclohexyl hydroperoxide. The third distillation column for cyclohexane has a top operating pressure of 0.05 MPaA, a top operating temperature of 59°C, a bottom operating pressure of 0.06 MPaA, and a bottom operating temperature of 68°C; and a secondary distillation column of the third stage distillation for cyclohexane has a bottom temperature of 70°C. The cyclohexane is evaporated by taking flash evaporation under reduced pressure, reaction heat, and a material heat exchange reboiler 08 as heat sources for the third distillation column 07. Gas-phase cyclohexane 071 in the third distillation column enters a condenser 14; a bottom liquid 072 in the third distillation column enters the secondary distillation column 10; and a steam reboiler 11 is used as a heat source for the secondary distillation column 10 to evaporate the cyclohexane, after which 95% of cyclohexyl hydroperoxide has been decomposed and the bottom liquid 100 (38,660 kg/h) in the secondary distillation column with the cyclohexane concentration about 60% is pressurized via a decomposition liquid discharge pump 12 and then delivered to a neutralization and saponification procedure.

A non-condensable gas 150 from the outlet of a vacuum pump 15 enters a tail gas absorption device, and a cyclohexane condensate 140 enters the cyclohexane-water separator 16.

Part of the cyclohexane from the cyclohexane-water separator 16 refluxes, the remaining cyclohexane 170 (215,320 kg/h) is pressurized by a cold cyclohexane pump 17 and then delivered to an oxidation heat recovery section to recover the cyclohexane from a tail gas, and the separated acid water 160 (1,500 kg/h) is delivered to an acid water treatment procedure.

(3) The decomposition liquid obtained from step (2) is subjected to water washing, followed by addition of an aqueous sodium hydroxide solution for a neutralization and saponification reaction, to convert the remaining cyclohexyl hydroperoxide into cyclohexanol and cyclohexanone completely.

The process water 2,000 kg/h and the decomposition liquids (38,660 kg/h ) obtained in the three-effect reaction distillation procedure for the oxidation liquid in step (2) are mixed and then separate out an acid water phase, followed by addition of an aqueous sodium hydroxide solution for a neutralization and saponification reaction in a neutralization and saponification reactor. After completion of the reaction, a water phase and an organic phase are separated from the saponification liquid via a saponification liquid separator; the water phase of the saponification liquid is discharged from the system, and the organic phase is delivered to a water washing column to wash off entrained alkaline water, and then delivered to a drying column to remove cyclohexane and water to obtain an alcohol-acetone mixture (15,464 kg/h) with the purity of more than 97%. The cyclohexane removed by drying the organic phase is circulated back to the non-catalytic oxidation procedure in step (1) to serve as a starting material for the non-catalytic oxidation in step (1).

In this example of the present invention, the decomposition reaction heat is effectively used to perform multi-effect reaction distillation, and the cyclohexane is oxidized to prepare the alcohol-acetone mixture with the purity of more than 97%, with the steam consumption of 35.8 t per hour and the converted steam unit consumption of 2.39 t per ton of alcohol-acetone mixture. Due to the elimination of the alkali decomposition procedure and the removal of most of acids resulting from the reactions by water washing, fresh alkali is only used in the neutralization and saponification procedure, such that the 32% alkali unit consumption is reduced to 150 kg per ton of alcohol-acetone mixture, and the cyclohexane unit consumption is 970 kg per ton of alcohol-acetone mixture.

### Comparative Example 1

A currently operating production line (120,000 tons/year) of cyclohexane oxidation plant combining homogeneous decomposition and heterogeneous decomposition includes the operation steps as follows.

Fresh cyclohexane (14,700 kg/h), circulating cyclohexane (511,912 kg/h) and compressed air (20,600 Nm³/h) with the molar fraction of oxygen being 21% are fed to an oxidation reactor for non-catalytic oxidation. The reaction temperature is controlled to 165°C-170°C, the reaction pressure is controlled to 1.32 MPaA, the gas-phase discharge from the top of the oxidation reactor is controlled at 110,954 kg/h, the cyclohexane oxidation liquid discharged from the bottom is controlled at 421,111 kg/h, and the concentrations of cyclohexyl hydroperoxide, cyclohexanol, cyclohexanone, cyclohexane and other acid and ester impurities in the oxidation liquid are 3.2%wt, 0.68%wt, 0.34%wt, 95.36%wt, and 0.42%wt, respectively.

The oxidation liquid (421,111 kg/h) at 165°C from the non-catalytic cyclohexane oxidation reactor exchanges heat with the decomposition liquid to reach 130°C, is then mixed with the decomposition catalyst tert-butyl chromate (1.8 kg/h) and the scale inhibitor 1-hydroxyethylidene-1,1-diphosphonate 011 (3.6 kg/h), and enters a homogeneous decomposition reactor for decomposition of about 80% of cyclohexyl hydroperoxide. At the same time, the cyclohexane (about 150,000 kg/h) is evaporated with the aid of flash evaporation under reduced pressure, reaction heat, and steam, and circulated back to the non-catalytic oxidation reactor; and the homogeneous decomposition liquid (269,711 kg/h) enters a heterogeneous decomposition procedure. The remaining 20% of the cyclohexyl hydroperoxide is decomposed in an aqueous sodium hydroxide solution, and then enters a waste alkali liquid separation procedure for two phase separation. The organic phase (269,711 kg/h) exchanges heat with the oxidation liquid to reach 155°C and then enters a cyclohexane recovery procedure, cyclohexane (245,338 kg/h) recovered using steam in a multi-effect distillation procedure is circulated to the non-catalytic oxidation reactor, and a crude alcohol-acetone mixture (23,080 kg/h) containing 33% of cyclohexane enters a crude alcohol-acetone mixture refining procedure.

The crude alcohol-acetone mixture (23,080 kg/h) containing 33% of cyclohexane from the cyclohexane recovery procedure reacts with an aqueous sodium hydroxide solution in a refining reactor, and is then separated by a saponification liquid separator to obtain an organic phase; and the organic phase is washed in a water washing column to remove the entrained alkaline water, and then fed to a drying column to remove cyclohexane and water to obtain an alcohol-acetone mixture (15,464 kg/h) with the purity of more than 97%.

An alcohol-acetone mixture with the purity of more than 97% is prepared by a cyclohexane oxidation process combining homogeneous decomposition and heterogeneous decomposition, with the steam consumption of 51.8 t per hour, the converted steam unit consumption of 3.45 t per ton of alcohol-acetone mixture, and the 32% alkali unit consumption of 260 kg per ton of alcohol-acetone mixture. The cyclohexane unit consumption is 980 kg per ton of alcohol-acetone mixture.

### Comparative Example 2

A currently operating cyclohexane oxidation plant (100,000 tons/year) with only heterogeneous alkali decomposition includes the operation steps as follows.

Fresh cyclohexane (12,750 kg/h), circulating cyclohexane (441,884 kg/h) and compressed air (17,166 Nm³/h) with the molar fraction of oxygen being 21% are fed to an oxidation reaction device for non-catalytic oxidation. The reaction temperature is controlled to 163°C-170°C, the reaction pressure is controlled to 1.32 MPaA, the gas-phase discharge from the top of the oxidation reaction device is controlled at 90,348 kg/h, and the cyclohexane oxidation liquid discharged at the bottom is controlled at 368,830 kg/h.

The oxidation liquid (368,830 kg/h) at 165°C from the non-catalytic cyclohexane oxidation reactor exchanges heat with the decomposition liquid, is then cooled with circulating water to reach 60°C, enters an alkali decomposition reactor, undergoes a cyclohexyl hydroperoxide decomposition reaction in an aqueous sodium hydroxide solution, and then enters a waste alkali liquid separation procedure for two phase separation. The organic phase (368,830 kg/h) enters a multi-effect distillation cyclohexane recovery procedure, cyclohexane (350,420 kg/h) is evaporated with the auxiliary heat from steam and then circulated to the non-catalytic oxidation reactor, and a crude alcohol-acetone mixture (19,234 kg/h) containing 33% of cyclohexane enters a crude alcohol-acetone mixture refining procedure.

The crude alcohol-acetone mixture (19,234 kg/h) containing 33% of cyclohexane from the cyclohexane recovery procedure reacts with an aqueous sodium hydroxide solution in a refining reactor, and is then separated by a saponification liquid separator to obtain an organic phase; and the organic phase is washed in a water washing column to remove the included alkaline water, and then fed to a drying column to remove cyclohexane and water to obtain an alcohol-acetone mixture (12,887 kg/h) with the purity of more than 97%.

An alcohol-acetone mixture with the purity of more than 97% is prepared by a cyclohexane oxidation process including heterogeneous alkali decomposition only, with the steam consumption of 56.3 t per hour, the converted steam unit consumption of 4.50 t per ton of alcohol-acetone mixture, the 32% alkali unit consumption of 450 kg per ton of alcohol-acetone mixture, and the cyclohexane unit consumption of 1,020 kg per ton of alcohol-acetone mixture.

| Type of production plant | Inventive Example | Comparative Example 1 | Comparative Example 2 |
|---|---|---|---|
| Converted steam unit consumption (t) per ton of alcohol-acetone mixture | 2.39 | 3.45 | 4.50 |
| Converted 32% alkali unit consumption (kg) per ton of alcohol-acetone mixture | 150 | 260 | 450 |
| Converted unit cyclohexane consumption (kg) per ton of alcohol-acetone mixture | 970 | 980 | 1020 |

## Claims

1. A method for producing cyclohexanone and cyclohexanol by oxidizing cyclohexane, comprising the steps of:
(1) oxidizing cyclohexane with molecular oxygen under non-catalytic conditions to obtain an oxidation liquid containing cyclohexyl hydroperoxide,
wherein oxidizing under the non-catalytic conditions comprises subjecting liquid cyclohexane and the molecular oxygen to an oxidation reaction in an oxidation reaction device without addition of a catalyst, to generate the oxidation liquid with an intermediate cyclohexyl hydroperoxide as a main component;
(2) adding a cyclohexyl hydroperoxide decomposition catalyst and a scale inhibitor to the obtained oxidation liquid from step (1) for multi-effect reaction distillation, to decompose more than 95% of the cyclohexyl hydroperoxide into cyclohexanol and cyclohexanone, and at the same time, to recover the cyclohexane unreacted in step (1) during the distillation and return the cyclohexane to step (1) to serve as a starting material for the oxidation reaction in step (1),
wherein the multi-effect reaction distillation of the oxidation liquid comprises: subjecting the oxidation liquid containing the cyclohexyl hydroperoxide to a decomposition reaction in a distillation column under actions of the decomposition catalyst and the scale inhibitor, and at the same time, condensing and circulating most of the cyclohexane unoxidized in step (1) from a top of the distillation column to an oxidation reactor in step (1) by means of multi-effect distillation using sensible heat of the oxidation liquid, decomposition heat and auxiliary heat, while discharging a small amount of acid water, and a bottom discharge after distillation comprises a decomposition liquid containing 50%-70% of cyclohexane, cyclohexanone, cyclohexanol and a trace amount of cyclohexyl hydroperoxide; and
(3) adding an alkali liquid and water to the decomposition liquid containing the 50%-70% of cyclohexane, as obtained from step (2), for a neutralization and saponification reaction, to convert the remaining cyclohexyl hydroperoxide completely into cyclohexanol and cyclohexanone to obtain an alcohol-ketone mixture,
wherein the neutralization and saponification comprises: removing most of acids from the decomposition liquid containing the 50%-70% of cyclohexane by water washing to separate acid water, treating an organic phase using the alkali liquid to neutralize acids and saponify esters in a crude alcohol-ketone mixture and to convert the remaining cyclohexyl hydroperoxide completely into cyclohexanol and cyclohexanone, separating a water phase from a saponification liquid, and then drying an organic phase of the saponification liquid to remove cyclohexane and water to obtain a mixture of cyclohexanone and cyclohexanol, and the cyclohexane removed by drying the organic phase is returned to step (1) to serve as a starting material for the oxidation reaction in step (1).

2. The method for producing cyclohexanone and cyclohexanol by oxidizing cyclohexane according to claim 1, wherein in step (1), the oxidation reaction device is formed by connecting no fewer than three oxidation reactors in series; and a reaction temperature is controlled to 160°C-170°C, and a molar conversion rate of the cyclohexane is controlled to 2.5%-4.0%, preferably 3.0-3.5%.

3. The method for producing cyclohexanone and cyclohexanol by oxidizing cyclohexane according to claim 1 or 2, wherein in step (2), the cyclohexyl hydroperoxide decomposition catalyst is an oil-soluble transition metal catalyst, preferably a transition metal salt catalyst or transition metal acid ester compound of chromium, cobalt, iron, manganese, molybdenum, vanadium or the like.

4. The method for producing cyclohexanone and cyclohexanol by oxidizing cyclohexane according to claim 3, wherein the oil-soluble transition metal acid ester compound is tert-butyl chromate.

5. The method for producing cyclohexanone and cyclohexanol by oxidizing cyclohexane according to one of claims 1 to 4, wherein in step (2), during the distillation, the cyclohexyl hydroperoxide undergoes a catalytic decomposition reaction, from which reaction heat is used to evaporate the cyclohexane, and a decomposition rate of the cyclohexyl hydroperoxide is controlled to 90%-98%, preferably 95%-97%.

6. The method for producing cyclohexanone and cyclohexanol by oxidizing cyclohexane according to one of claims 1 to 5, wherein in step (2), the scale inhibitor is an HEDP ester, i.e., 1-hydroxyethylidene-1,1-diphosphonate ester.

7. The method for producing cyclohexanone and cyclohexanol by oxidizing cyclohexane according to one of claims 1 to 6, wherein in step (2), the multi-effect reaction distillation of the oxidation liquid is performed in a multi-stage distillation column, preferably for three-stage distillation, in which case a first-stage distillation column has a top operating pressure of 0.20 MpaA-0.35 MpaA, a top operating temperature of 105°C-130°C, a bottom operating pressure of 0.22 MPaA-0.4 MPaA, and a bottom operating temperature of 110°C-135°C; a second-stage distillation column has a top operating pressure of 0.10 MpaA-0.24 MpaA, a top operating temperature of 85°C-110°C, a bottom operating pressure of 0.12 MPaA-0.25 MPaA, and a bottom operating temperature of 90°C-105°C; and a third-stage distillation column has a top operating pressure of 0.04 MpaA-0.06 MpaA, a top operating temperature of 55°C-70°C, a bottom operating pressure of 0.05 MPaA-0.07 MPaA, and a bottom operating temperature of 65°C-80°C, and the decomposition liquid discharged from a bottom enters the second-stage distillation column for the three-stage distillation and is fine-adjusted using a small amount of vapor to a cyclohexane content of 50%-70%, preferably 60%-65%.

8. The method for producing cyclohexanone and cyclohexanol by oxidizing cyclohexane according to claim 1, wherein in step (3), the alkali liquid is an aqueous NaOH solution; and a neutralization and saponification process comprises: washing the decomposition liquid with water, subjecting the water-washed decomposition liquid and the aqueous NaOH solution to a neutralization and saponification reaction in a reactor, separating a saponification liquid, removing cyclohexane and water in a drying column to obtain an alcohol-ketone mixture with a purity of more than 97%.

9. The method for producing cyclohexanone and cyclohexanol by oxidizing cyclohexane according to one of claims 1 to 8, wherein in the neutralization and saponification process, an ion concentration of OH- in the alkali liquid is 0.3 Mol/L-1.5 Mol/L, preferably 0.5 Mol/L-1.0 Mol/L.

10. The method for producing cyclohexanone and cyclohexanol by oxidizing cyclohexane according to one of claims 1 to 9, wherein in step (3), a reaction temperature for the neutralization and saponification is controlled to 80°C-110°C, preferably 90°C-100°C.
